# EUROPEAN PATENT SPECIFICATION

(11) **EP 2 242 518 B1**
(45) Date of publication and mention of the grant of the patent: **29.06.2016**
(21) Application number: 08856170.9
(22) Date of filing: 05.12.2008
(51) Int. Cl.: A61L 2/18, A61L 2/23, A61L 9/01, A01N 59/02, C02F 1/50

(54) **DEODORIZING AND SANITIZING COMPOSITIONS**
DESODORISIERENDE UND DESINFIZIERENDE ZUSAMMENSETZUNGEN
COMPOSITIONS DÉSODORISANTES ET DÉSINFECTANTES

(30) Priority: 07.12.2007 WO PCT/IT2007/000857
(43) Date of publication of application: 27.10.2010
(73) Proprietor: Manna, Emanuela, 00137 Roma (IT); Santarelli, Maria Teresa, 00137 Roma (IT)
(72) Inventor: Manna, Emanuela, 00137 Roma (IT); Santarelli, Maria Teresa, 00137 Roma (IT)
(74) Representative: Raimondi, Adriana
(86) International application number: PCT/EP2008/066892
(87) International publication number: WO 2009/071664

(56) References cited:
- WO-A-96/14092
- US-A- 4 230 478
- US-A- 4 781 854
- US-A- 5 559 089
- US-A- 5 679 661
- US-A1- 2003 070 692
- US-A1- 2003 070 696
- US-A1- 2003 215 441
- US-A1- 2006 039 841
- US-A1- 2006 088 498
- US-B1- 6 448 062

## Description

### Field of the invention

The present disclosure relates to monopersulfate-based deodorizing and sanitizing compositions. The disclosure also relates to their preparation and formulation in kits and their use for neutralising bad-smelling substances and toxic gases and for sanitization.

### Background art

The problem of the emission of bad odours is very extensive and embraces numerous categories of compounds present in liquid, solid or gaseous matrices. The difficulty of their elimination is related to the physical state of the material emitting the odour, and therefore multiple products have been prepared for the various different applications. Nevertheless, to date, no simple, easily producible compositions are available from the industrial point of view that are capable of deodorizing and disinfecting the various types of materials.

Control of odours is a very great problem, given the substantial variety of volatile substances responsible for the odours and the very considerable numbers of processes that generate them, often deriving from the activity of bacteria, particularly anaerobes, that give rise to bad-smelling and often toxic reduction products. The problem is particularly evident on livestock breeding farms where it takes on very notable proportions, but also in sanitary and industrial environments as well as in domestic ones, due to the steadily increasing presence of domestic animals of various types. Furthermore, the control and removal of bad-smelling liquid and solid materials and gases is of particular interest in the context of environments and machinery related to urban waste collection and disposal activities.

There are also problems related to the disposal of animal excreta present in the home for which, for example, sizeable specific litters are used that absorb or are mixed with the excreta and then have to be disposed of. The control of bad-smelling emissions is therefore a particularly strongly perceived problem and one which currently has not been resolved in a satisfactory manner and is believed to require the availability of products obtained with economic, low-toxicity formulations for users and the environment, products which are also endowed with antibacterial activity and are easily manageable by inexpert people such as elderly subjects and children.

It should be recalled, moreover, that bad-smelling substances generally also have a high microbial load and therefore, necessarily, the formulations to be used should be simultaneously deodorizing and disinfecting, or at least such as to lower the microbial load of the materials treated.

Most of the known methods use oxidizing compositions containing fragrances to eliminate the bad odours, which, however, act only by masking them, superimposing themselves upon them. In US 4230478 a deodorizing composition is made up of an oxygen carrying element associated with a synergistic odour masking compound. Among the oxygen releasing components, only H₂O₂ is exemplified, always in combination with aromatic essences deriving from pine oils (terpenes). In WO 2007/019298 compositions for oxidizing organic compounds in pool or spa water are described containing an alkali metal monopersulfate in combination with a fragrance. It is stated that the monopersulfates, when used as shocking agents, take time to reduce the odour-causing organic materials in the water.

There are also known (US4781854, US5679661, WO96/014092, US2003/070692, US2003/070696, US2006/039841, US2006/088498) complex formulations containing peroxides in combination with zeolites, surfactants, builders, buffers, dyes, etc. to which compounds may be added, that are capable of reacting with the bad-smelling volatile substances, such as enzymes, bacteria, metal transition complexes or amphoteric products added with antibacterial substances, but these formulations are generally complex, sometimes risky to use and often expensive. Such compositions are in general used for disinfection, sanitization, bleaching, cleaning and may also contain perfumes to deodorize.

Most bad-smelling substances belong to the following categories:
- volatile compounds of an acid nature, such as sulphurated hydrogen and other sulphur-based compounds, including the mercaptans;
- volatile compounds of an alkaline nature, such as ammonia and amines and, more generally, compounds deriving from decomposition/putrefaction processes;
- aldehydes and ketones and, more generally, organic compounds more or less emitting disagreeable odours.

The problem of deodorization has been addressed in WO2006/137821, preparing liquid mixtures based on cocamidopropyl betaine, an amphoteric surfactant to which can be added solvents, acids and salts, e.g. salts of ethoxy alcohols, such as, for example, sodium lauryl ethoxysulphate. To the mixtures are added a stabilising agent, citric acid, dihydrated sodium citrate and water. Other components can also be added as fragrances. Particular attention must be paid in their preparation in order to control the viscosity which must not exceed the critical values for the purposes of their use, for example as sprays, acting also on the temperature. The mixtures are modified as a function of the type of substrate to be treated and are capable of forming complexes with the gas molecules, neutralising a very extensive range of inorganic, acidic and basic compounds, as well as organic substances such as aldehydes, carboxylic acids and amines. The mixtures are also capable of exerting a bactericidal action by adding Triclosan, a bacteriostatic agent active against Gram-positive and Gram-negative bacteria. However, attention should be drawn to the complexity of the preparation of the mixtures described in WO2006/137821, the modifications needed to be made to them for better application in the wide range of substrates, and the flocculating capacity of the materials in suspension, which is not always useful. In fact, in the case of suspended organic materials, their partial prior decomposition would be useful with clarification of the matrix to allow better chemical attack by the bacterial load present (e.g. animal excreta). Moreover, their disinfectant action requires the addition of a further compound to the basic mixture.

Compounds and formulations based on oxidising agents can be used in the case of bad-smelling products of a reducing nature (e.g. sulphurated hydrogen, ammonia) and also many organic substances can be oxidised (e.g. aldehydes, ketones, amines) and thus peroxides of various types are often present in the formulations. The high reactivity of oxidants, however, generally makes their aqueous solutions poorly stable and thus the formulations in aqueous solution often require the addition of stabilising substances, chelants of metals that catalyse the redox reactions or pH modification, often in a distinctly acidic direction. Among the known oxidants, the peroxides are often used, and especially the persulphates owing to their substantial oxidation potential which makes them particularly reactive towards many substances.

In US 6,028,045 a study is reported which revealed that the aqueous solutions of the persulphates are poorly stable, particularly if the pH value is 2 or more and if substances are present that catalyse their decomposition. pH was therefore blocked between 0 and 0.9 by means of the addition of methanesulphonic or sulphuric acid to maintain it between 0.2 and 0.7 and free radical blockers (radical scavengers) were added (e.g. dihydroxybenzenes and their analogues) responsible for catalysing the decomposition of peroxides, also using deionised water so as not have in solution the cations present in drinking water, which are catalysts of decomposition. To the solutions is added preferably an emulsion of two non-ionic detergents as additional stabilisers.

The stability tests of the solutions at pH 0.2 or 0.5 revealed a duration of 12 weeks without any loss of oxidising power, while no stability tests over lengthier periods were reported. The persulphate preferably used is potassium peroxymonosulphate (potassium monopersulphate triple salt) present in a composition known as Caro's reagent. The applications reported relate to laundries, household cleaning, and particularly toilet cleaning, whereas no mention is made of any deodorizing capacity.

The stability of these solutions is unquestionably quite long-lasting if we bear in mind that the persulphates in aqueous solution lose more than 50% of their oxidising power within the space of one week, but the stability invariably proves limited for a large-scale application. There are, moreover, other limitations in terms of application deriving from the distinctly acidic pH, which is too aggressive for various types of materials, and from the need to use deionised water for the preparation.

An application of peroxymonosulphate in aqueous solution for the treatment of the liquid phase of manure and for neutralising the toxic gases generated by it is reported in US 4,160,656. Various peroxides were tested, such as peracetic acid, which, however, leaves a strong acetic acid odour, and hydrogen peroxide at pH 4-8, which decomposes too quickly. Formaldehyde alone has yielded no results, whereas, when combined with hypochlorite, it yields excellent deodorization, but has been discarded both because the two components need to be added separately to the liquid manure and because toxic chlorine dioxide may be formed. Formaldehyde was therefore combined with various oxidants such as hydrogen peroxide and peroxymonosulphate, which enable a single solution to be prepared by blending the two components immediately prior to use. The drawbacks of this application stem from the irritant action of formaldehyde, as disclosed by the authors themselves, from the use of poorly stable aqueous solutions and from the modalities of use of the mixture which involves the preparation of two separate solutions to be blended immediately prior to use. Pertaining to the context of solid formulations of disinfectants are the compositions described in US 4,822,512, containing peroxymonosulphate in triple salt form (2KHSO₅.KHSO₄.K₂SO₄ Caroat® or Caro's reagent or Oxone®) capable of oxidising the sodium chloride added and allowing the development of chlorine in solution. Also present in the compositions are sulphamic acid, a blocker of the chlorine developed, sodium hexamethaphosphate as a pH buffer and metal chelant, malic acid and sodium dodecylbenzenesulphonate as a detergent. The action of the preparations is bactericidal and virucidal and their application is in the field of detergents and disinfectants for surfaces and objects.

The need for a pH of around 2 in the solution to allow better development of chlorine, however, makes the solutions aggressive towards many materials, bearing in mind also the presence of a further powerful oxidant such as peroxymonosulphate. Moreover, the presence of chlorine does not make the preparations suitable for environmental applications, given the possibility of the formation of potentially toxic organic chlorine derivatives.

Other solid formulations based on peroxymonosulphate triple salt, in combination with other components to obtain granules or tablets for dish-washers are described in US 5,559,089. Peroxydisulphate failed to yield good results unlike peroxy-monosulphate, which was assayed in the form of a salt with various cations, including organic cations. The mixtures prepared are very complex owing to the needs deriving from the particular application and entail the presence not only of Oxone®, but also of enzymes, detergents, organic dispersing agents, agents for bringing pH into the alkaline range, agents for the removal of chlorine, organic emulsifying agents, organic sequestering agents containing nitrogen, anionic and non-anionic surfactants with a low foam content.

In the state of prior art the deodorizing activity of Oxone® or of other monopersulfate-compounds has never been reported. The compositions disclosed in the art are basically aimed at achieving a bleaching and/or antibacterial, antiviral and disinfecting action without any particular or specific deodorizing action, except when in combination with perfumed compounds.

There was therefore a strongly perceived need for the availability of compositions with an effective deodorizing and sanitizing action capable of overcoming the problems mentioned above.

In the description here below percentages are expressed throughout as percentages by weight, unless otherwise specified.

### Summary of the invention

It is an object of the present invention the use of monopersulfate-compounds not in combination with any fragrance or odorous substance to deodorize solid and liquid bad smelling matrices, i.e. manufactured objects or substrates such as manure or waste. Advantageously such monopersulfates are used in combinations with surfactants and possibly with zeolites.

Another object is the use of a deodorizing and sanitizing composition comprising a basic mixture of components (a+b) in which the a:b ratio ranges from 1:4 to 4:1. Preferably the composition comprises:
a) a peroximonosulfate-compound in amounts ranging from 20 to 80% of (a+b);
b) a surfactant (selected from the group consisting of anionic, cationic and non-ionic surfactants) in amounts ranging from 80 to 20% of (a+b);
c) an alkalinising agent (selected from the group consisting of water-soluble salts and bases) suitable for producing a pH in water ranging from 1.5 to 8.5 when in the presence of the other components of the mixture, preferably pH ranging between 2.0-7.5, more preferably from 2.5 to 7.0, and even more preferably around 4.5-7.0. Preferred agents are, for example, buffer substances such as the bicarbonates, added in amounts ranging from 0 to 20% of the basic mixture (a+b), preferably 0.05-0.15%;
d) natural or synthetic zeolites, preferably clinoptylolite and/or zeolite A, added in amounts ranging from 0 to 50%, preferably from 10% to 40% of the basic mixture (a+b);
e) oxidimetric colouring agents, in amounts ranging from 0 to 0.01%, preferably 0.001-0.003% of the basic mixture (a+b).

Said basic composition (a+b) is characterized in that when placed in an aqueous medium containing a mixture of aerobic and anaerobic bacteria produces a selective removal of anaerobic ones and a decrease in the BOD₅ value of the mixture during a period of 3-5 days and a subsequent increase of said value during a period of the following 6-8 days.

Also disclosed is the process for the treatment of materials and/or environments comprising the step of mixing the composition of the invention (in the liquid or solid state) with the material to be treated or spraying or nebulising the liquid composition as an aerosol in the environment to be treated.

Further objects will be evident from the following detailed description of the invention.

### Brief description of the figures

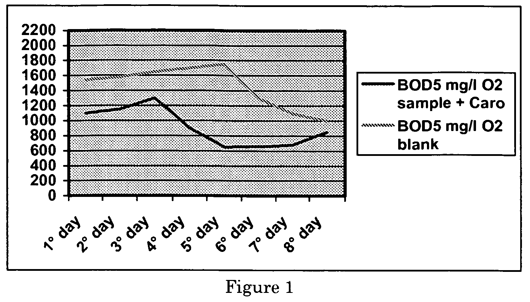

Figure 1: Trend of BOD₅ in an acqueous medium containing aerobic and anaerobic bacteria (pig excreta). BOD₅ measures the rate of oxygen uptake by micro-organisms in a sample. The BOD₅ measurement was monitored continuously and a graph of its evolution was plotted by using the method described in Standard Methods of the examination of Water and Wastewater APHA-AWWA-WPCF.

### Detailed description of the invention

The invention is based on the founding that monopersulfate-compounds neutralize bad-smelling liquid and solid matrices in a quick and quite surprising manner without the co-presence of fragrances or of other additional substances. They only act alone or in synergistic combination with surfactants and possibly with silicoaluminates in zeolitic form.

Within the scope of the present invention the term "to disinfect" means to "destroy all the possible microbes of any type present in an inert object", whereas what is meant by the expression "to sanitize" is to "render physically clean and substantially reduce, in the best and most practical way, agents that may prove damaging to health".

In the present invention the terms monopersulfate and peroxymonosulfate are considered synonyms and are intended to identify the HSO₅⁽⁻⁾ anion. In the present invention the terms monopersulfate-compound(s) and peroxymonosulfate-compound(s) are considered synonyms and are intended to identify compounds characterized by the presence of the couple H₂O₂/HSO₅⁽⁻⁾.

The monopersulfate-compounds are well known compounds such as potassium, sodium and magnesium salts, as well as binary and ternary mixed salts of monopersulfate with alkali metal sulfates. Peroxidisulfates are excluded from the scope of the present invention in that they are less effective than peroxymonosulfates. One such salt, sold as Oxone®, is well known and described in the literature as a mixture of potassium monopersulfate with potassium sulphate and potassium bisulfate, or as a "triple salt". Methods for preparing monopersulfate compositions containing the triple salt 2KHSO₅ ·KHSO₄ ·K₂SO₄ are described in US3041139 and WO2005/030648.

In the present invention with the terms bad smelling substances are intended:
- solid or liquid matrices of various natures which produce unpleasant odours such as: excreta, solid urban waste (SUW), rubbish or waste dump percolate, industrial processing waters in general, particularly those treating animal waste residues deriving from the processing of the meat of pigs, cattle, sheep, chickens or other animals, and, more generally, compounds deriving from decomposition/putrefaction processes, tannery waters, industrial effluent waters;
- containers and manufactured objects in contact with said solid or liquid matrices;
- urban areas contaminated by said solid or liquid matrices such as sea harbours or roads and alleys; bad-smelling gases produced by industries or by factory farming.

Bad smelling in liquid effluents or solid matrices is mainly caused by the presence of anaerobic bacteria. It can be determined by using different methods such as the use of colorimetric detection and/or testing by means of the olfactory sensory system of a number of subjects. Both methods are illustrated herein below.

The deodorizing action of the compositions according to the invention has been found to derive from the synergistic effect of the species originating from the dissolution/contact of peroximonusulfate-compounds with water, for example the water contained in the matrices to be treated. The deodorizing effect is the result of a series of reactions, the first of which is an equilibrium reaction in which the couple H₂O₂/HSO₅⁽⁻⁾ acts according to the following reaction scheme:

HSO₅⁽⁻⁾ + H₂O H₂O₂ + SO₄⁽²⁻⁾ + H⁺ (I)

Then further reactions follow, in which the HSO₅⁽⁻⁾ species is mainly involved. Owing to the complexity of the environment in which the HSO₅⁽⁻⁾ species is made to act (bad-smelling substrates containing, among others, sulfides, mercaptans, ammonia, amines, etc.) it was not evident from the standard redox potential of the HSO₅⁽⁻⁾ species (known in literature, E_{0HSO5(-)/HSO4(-)}=1.44 eV) that the reducing substances causing bad-smelling could be completely oxidized thus producing non-bad-smelling substances such as nitrates and sulfates. According to (I), the mechanism of action of the composition of the invention is based on the assumption that peroximonosulfate ions in aqueous solution react with water giving origin to hydrogen peroxide until an equilibrium is reached that is function of the respective redox potentials of the two oxidant co-existing species: H₂O₂ and HSO₅⁽⁻⁾. The deodorizing action is therefore performed in two main steps:
- the first one is quite immediate and consists in the oxidation reaction of reducing substances (sulphur compounds, H₂S, amines, ammonia) by the hydrogen peroxide/peroxymonosulfate couple; The bad-smelling substances are oxidized to less or non-smelling compounds, such as nitrates and sulfates.
- the second one is long lasting and is due to the selective antibacterial activity of the oxidizing couple H₂O₂/HSO₅⁽⁻⁾ against more sensitive to oxidants anaerobic bacteria (gram-positive and negative) responsible of the production of the above mentioned reducing substances. The deodorizing action is therefore particularly effective in that bad smelling substances which are destroyed together with the bacteria responsible for their production Furthermore when peroxymonosulfate comes in contact with the aqueous medium produces an immediate and localized amount of oxygen available to the aerobic bacteria for their activity against the organic substances present in the medium to be deodorized. Said action can be shown by the BOD₅ curve and is useful for the oxidation of the remaining organic materials (see the experimental part).

As an additional effect, the couple H₂O₂/HSO₅⁽⁻⁾ acts in synergy with surfactants with the following mechanism. Surfactants modify the strucuture of the cell wall thus favouring penetration of active oxygen radicals and of the HSO₅⁽⁻⁾ species inside the cells and producing the Haber Will Statter reaction with chlorides in cytoplasm. So HSO₅⁽⁻⁾, besides acting as an oxydant with respect to microorganisms, acts on chloride intra- and extra-cellular components to produce the chlorinated toxic *species in situ.* Anaerobic bacteria are more sensitive of aerobic ones to these actions and this mixture destroys them selectively allowing the aerobic bacteria to continue their activity against the remaining organic materials. This selectivity derives from the synergistic activity of surfactants and oxidant compounds which allows to use low concentrations of the mixture.

The disclosure relates to a solid or liquid composition with a broad spectrum of applications, capable of eliminating bad-smelling odours from solid or liquid matrices as mentioned in the above. It is also capable of exerting a sanitizing action. The applications may extend to domestic uses, given the simplicity of use and low toxicity of the formulations. The mixture is characterised by the presence of an oxidant (peroxymonosulphate), by a surfactant and possibly by a zeolite and a colouring agent can be advantageously added which performs the function of indicating the activity of the composition when formulated as an aqueous mixture.

In more detail the present composition includes monopersulfates with any compatible cation. Compatible cations are tipically: alkali metal cations, such as sodium or potassium; alkaline earth cations, such as calcium or magnesium; quaternary ammonium cations, such as tetraalkylammonium.

Preferred monopersulfates are selected from the group consisting of sodium monopersulfate, potassium monopersulfate, calcium monopersulfate, magnesium monopersulfate, tetraalkilammonium monopersulfate, complex monopersulfate salts such as Oxone®, and mixtures thereof. More preferred is the monopersulfate triple salt 2KHSO₅ ·KHSO₄ ·K₂SO₄ and highly preferred is the stable species produced under the trademarks of Oxone® and Caroat®.

Peroxymonosulfates are preferably used in amounts higher than 0,024 g (0,0066 g in terms of Oxone®) in 100 ml to deodorize liquid matrices or liquids containing suspended particles and preferably between 0,024-0,24 g (0,066-0,66 g in terms of Oxone®) in 100 ml, added directly to them as solid. Peroximonosulfates are used in solution to deodorize solid materials in amounts higher than 0,0024 % (0,0066 % in terms of Oxone®) and preferably in concentrations between 0,024-0,24 % (0,066-0,66 in terms of Oxone®) using 1 l of solution for 1 m³ of materials (ex. solid urban wastes) or 1 l for about 100 m² of surface treated by a spraying system. The amount of solution used may be varied in function of the washing system used.

In comparison with other peroxy compounds, the peroxymonosulphates possess the great advantage of transforming bad smelling substances into products (such as sulphates and nitrates) which are not toxic either for living beings or for the environment.

The anionic surfactants are preferably selected from the group consisting of alkylsulphonate and alkylarylsulphonate salts, alkylsulphate and alkylarylsulphate salts, alkylnaphthalene-sulphonate and alkyldiphenylsulphonate salts, and dialkylsulphosuccinate salts.

Particularly preferred are the alkylsulphate and alkylarylsulphate salts of sodium, potassium and ammonium; of the cations of protonated mono-, di- and tri-ethanolamines or protonated isopropylamines; the alkylnaphthalenesulfonate and alkyldiphenylsulphonate salts of sodium, potassium and ammonium; the dialkylsulphosuccinate salts of sodium, potassium and ammonium, of the cations of protonated mono-, di- e tri-ethanolamines or of protonated isopropylamines. Particularly preferred is sodium dodecylbenzenesulphonate.

The cationic surfactants are preferably selected from the group consisting of n-alkyldimethylbenzylammonium chloride, n-alkyldimethylethylbenzylammonium chloride, dialkyldimethyl-ammonium chloride, alkyloxypropyl-dihydroxyethy-lmethylammonium chloride, alkylbenzyl-imidazolyl chloride and the diquaternary cationic surfactants known as Diquat.

The non-ionic surfactants are preferably selected from the group consisting of ethoxylated nonylphenols, ethoxylated dinonylphenols, ethoxylated linear alcohols, ethoxylated dodecylphenols, ethoxylated octylphenols, alkanolamides, ethoxylated alkanolamides, ethylene oxide-propylene oxide copolymers, ethoxylated-propoxylated nonylphenols, ethoxylated-propoxylated linear alcohols.

The colouring agents that can be advantageously used in the compositions according to the invention are selected from the group consisting of all those substances capable of manifesting colour changes (preferably visually detectable) in the presence of a changing (decreasing) of the oxidising power of the composition. The amount to be added is a function of the intensity of colour one wishes to confer upon the final mixture, for the purposes of making the colour change more evident.

The colouring agents are preferably selected from the group consisting of oxidimetric indicators such as, for example: diphenylamine, diphenylbenzidine, indigo derivatives, phenothiazines or safranines. Particularly preferred is amaranth.

The zeolites are preferably selected from the group consisting of silicoalluminates of synthetic or natural origin. Among the natural zeolites, most preferred is clinoptylolite; among the synthetic zeolites, preferred are zeolite A, preferably activated (described in WO1999IB00757 and produced by PANACEO). Zeolites are added to the mixture of the invention to adsorb gas, metals, organic materials and catalyze oxidation reactions, further enhancing the deodorizing power of the composition.

The formulations in liquid form can be obtained by dissolving the various components a)-e) in water, typically at the time of use. The liquid formulations are stable; their activity is indicated by the colouring agent, the colouring intensity of which diminishes when the oxidising power drops below the desired limits, typically below 50% of the initial oxidising power.

The pH of the liquid compositions typically ranges from 2.0 to 2.5 and this type of formulation can be used as is in the case of applications on matrices that are not damaged by a distinctly acidic pH. In any event, the pH can be brought to neutrality by means of the addition of an alkalinising agent, for example a water-soluble salt or base, typically a buffering agent, such as, for example, a bicarbonate, generally potassium, sodium or ammonium bicarbonate The amounts vary as a function of the agent used. By way of an example, the amounts are typically 0.05 to 0.15% of (a+b) and the deodorizing and sanitizing capacity of the compositions is unaltered, thus allowing their use on more delicate and pH-sensitive matrices or for increasing their safety, as in the case of domestic uses.

According to a first embodiment of the invention, the individual components of the mixture are solid and are blended dry or are added to an aliquot of water adequate for achieving a final mixture of (a+b) according to the concentrations expressed above. According to an alternative embodiment, component b) is liquid and is blended with other solid components and the mixture is diluted in water until the desired final concentration of (a+b) is achieved.

Advantageously, the various components from a) to e) can be packaged in suitable formats (dosage units) containing the metered amounts to be used directly at the time of use for dissolving the component in the amount of water needed to produce the appropriate final concentration for the various types of applications.

The solid components can also be prepared in the form of granulates and tablets, which are very practical when used for applications on masses (for example, mixed with solid waste), for automatic metered dose systems or for small applications.

In order to check the safety of the compositions used in the invention, the following test was performed to verify the possibility of direct reactions between the peroxide and the organic substances in the blending operation needed for the preparation of the mixtures. The test was carried out by means of thermal analysis of the individual components and the mixture. Sodium lauryl sulphate presents a fusion endotherm at 80-100°C and exothermal decomposition at temperatures above 200°C. Caro's reagent presents an exothermal decomposition at 140-200°C (peaking at 182°C) with great development of heat. Analysis of a mixture consisting of 33% sodium lauryl sulphate and 66% Caro's reagent presents a first fusion at 110-120°C (peaking at 115.8°C) followed at 140-200°C by a decomposition attributable to the decomposition of the Caro's reagent present in the mixture. Impact tests were performed to determine the mechanical impact sensitivity corresponding to the energy of 300 kg/cm² without any changes occurring in the mixture. This trend demonstrates that the presence of the surfactant has had no effect on the peroxide decomposition process and confirms the absence of any risk in the preparation of solid compositions and the substantial stability of the compositions even at high temperatures and in response to impact.

The mixtures used in the present invention, if used at the concentrations indicated above (0.05-10% in terms of a+b), are capable of acting selectively only on the anaerobic part of the bacterial load present in the matrix to be treated, leaving the aerobic part unaltered and capable of continuing its attack on the organic materials. Tests of antibacterial activity carried out on anaerobic (*Actinomices israeliti*) and aerobic (*Esclaerichia coli*) bacteria evidence this selectivity. A broth-culture in Muller Hinton broth (M.H.B.-Oxid-) was prepared having a bacterial concentration of 5 x 10⁶ CFU/ml, pH=6. The decrease of bacterial charge produced by a water solution of HSO₅⁽⁻⁾ 0,24 % (Oxone® 0,66%) and its dilutions 1:2 and 10⁻² on the inoculum are shown in the following Table 1 in terms of residual bacterial charge measured as optical density O.D. at 450m.
In order to show the synergistic effect of the peroxymonosulfate and surfactants (a+b), a mixture of HSO₅⁽⁻⁾ 0,24 % (Oxone® 0,66%)/Sodium laurylsulfate 0,33% was tested at a dilution of 10⁻². As evident from Table 1, a decrease of 10³ in the amount of anaerobic bacteria at 30" was observed with respect to the same solution without surfactant, thus proving the synergistic effect.

**Table 1**

| Decrease of bacterial charge produced by a solution of HSO₅⁽⁻⁾ 0,24 % (Oxone 0,66%) and its dilutions 1:2 and 10⁻² on an inoculum of 5 x 10⁶ CFU/ml | | | |
|---|---|---|---|
| Concentration | Contact times | *Actinomices israeliti** | *Escherichia coli** |
| 0,24 % | 30" | 0 | 5 x 10⁶ |
| | 4' | 0 | 5x10⁶ |
| | 15' | 0 | 5x10⁵ |
| | 120' | 0 | 5 x 10⁴ |
| dil. 1:2 | 30" | 0 | 5 x 10⁶ |
| | 4' | 0 | 5 x 10⁶ |
| | 15' | 0 | 5 x 10⁵ |
| | 120' | 0 | 5 x 10⁴ |
| dil. 10⁻² | 30" | 5 x 10³ | 5 x 10⁶ |
| | 4' | 5 x 10² | 5 x 10⁶ |
| | 15' | 0 | 5 x 10⁶ |
| | 120' | 0 | 5 x 10⁵ |
| Mixture (a:b = 2:1; 0.01%) | 30" | 0 | 5 x 10⁶ |
| | 4' | 0 | 5 x 10⁶ |
| | 15' | 0 | 5 x 10⁶ |
| | 120' | 0 | 5 x 10⁵ |

| | | | |
|---|---|---|---|
| * Residual bacterial charge | | | |

The compositions used in the invention, whether used in the solid or even in the liquid state, manifest a broad spectrum of applications capable of eliminating the bad odours emitted by solid and liquid matrices of various natures.

The formulations can be prepared in kits ready for use and containing the following components in defined amounts, packaged singly or in blended mixtures, possibly dilutable in water:
a) an aliquot of peroximonosulfate-compound, in preferred amounts of 70,85-18,80 % HSO₅⁽⁻⁾;
b) an aliquot of surfactant, preferably 24-65 %;
c) possibly an aliquot of an alkalinising agent, preferably 0,1-1,0 %;
d) possibly an aliquot of zeolite, preferably 5-15 %;
e) possibly an aliquot of a colouring agent, preferably 0,05-02 %;
f) instructions for use.

The components of the composition can be in the form of a solid such as powder, flakes, granules, pellets, tablets, lozenges, pucks, bricks, briquettes, solid blocks, unit doses or other solid forms.

The compositions can be packaged in a variety of packages or packaging materials, such as, for example, a simple bottle or a jar, a unit dose tablet or block, a "tear and pour" pouch, or a water-soluble packet.

The composition can be dispensed by any of a variety of known methods, such as, for example, simply sprinkling or spreading the composition in any solid form onto the matrices to be treated, or dissolving such solid form into water. The thus obtained liquid forms of the composition according to the invention can be applied directly onto the area to be treated using, for example, a sprayer or an aerosol can. In industrial deodorizing plants the liquid forms can be applied directly to the counter-current washing towers or the solid forms can be dissolved in the aqueous washing liquids.

The formulations are easy to prepare, possess low toxicity, are practical to use, even in the domestic context and have a broad spectrum of action.

The compositions used in the present invention manifest a broad spectrum of action and are capable of deodorizing and sanitizing bad-smelling solid and liquid matrices of any origin, such as animal excreta, solid urban waste, rubbish dump and waste tip percolate, processing waters of industries that treat animal residues from the processing of the meat of pigs, cattle, sheep, chickens and other animals, and industrial effluent waters.

For uses in the context of SUW an a:b ratio of approximately 1:1 is preferable in order to increase the detergent capacity against organic materials. This mixture makes it possible to wash, deodorize and sanitize the means of transport of solid urban waste and liquid waste and can be applied in mechanical bin-washing devices to deodorize, wash and disinfect the bins and SUW containers, as well as household or food industry waste bins and containers.

The applications also extend to domestic uses, given the simplicity of use, the possibility of modifying pH so as to make it less aggressive and the low toxicity of the formulation. The uses extend to the deodorizing, cleansing and sanitizing of environments and surfaces, to the treatment of the organic component of waste so as to conserve it for several days prior to collection, in the deodorizing and disinfection of domestic animal litters, and in the washing, deodorizing and disinfection of floors and surfaces in general, particularly those of wallpapers and coverings, upholstery and household electrical appliances such as refrigerators, freezers, cooking plates, ovens, washing-machines, dish-washers, etc.

The compositions used in the present invention are very simple to use and can be applied as is in the solid phase by pouring the amount of powder necessary for the matrix to be treated or can be dissolved in the necessary amount of water and the solution obtained can be used for nebulisation and spraying on surfaces or environments or added directly to the matrix to be treated, be it a solid, liquid or suspension.

The components of the mixtures present very low toxicity, are non-irritant and do not generate allergies. The compositions are simple, very easy to use without any particular caution, not toxic for the environment because their components are transformed in salts usually present in the soils and waters. Furthermore they don't generate toxic or hazardous products reacting with other compounds, moreover are safe for humans and animals and totally eco-compatible.

The following examples are provided to illustrate the invention and are not to be regarded as limitative of the scope of the invention.

### Determination of available oxygen

The determination of the oxygen available for the various oxidising compounds is quite easily done using the most common standard analytical methods; for example, in the case of monopersulphate, also present in the triple salt (or Caro's reagent) the iodometric method was used.

The percentages of available oxygen released by the compositions according to the present invention as a function of pH from 2 to 7 vary from 1% to 3.5%, and are very different from those used for application in dish-washing machines (US 5,559,089), a greater oxygen availability being necessary to activate the oxidative processes of the oxidisable bad-smelling substances, cut down the anaerobic bacterial load and, finally, facilitate the aerobic bacteria in their processes of attacking the organic matrices.

### Determination of deodorizing action

The experimental methods used to determine the efficacy of the compositions were of two types, namely, the use of colorimetric detection and assay systems for bad-smelling and toxic substances in the matrices, and testing by means of the olfactory sensory system of a number of subjects.

Matrices of various natures were placed in suitable glass flasks, left at room temperature to simulate the normal conditions of usage of the compositions, connected up to the detection and assay system consisting of Dräger ampoules (US 4,160,656), for the determination of ammonia, sulphurated hydrogen, mercaptans, cyanhydric acid and amines, after washing the entire apparatus with a nitrogen current to eliminate any external interference. The values obtained (Table 2) fall within a broad range of concentrations as a function of the matrices analysed with high values in animal excreta, for example, pig excreta (H₂S 60-110 ppm; mercaptans 150-350 ppm; NH₃ 60-100 ppm; HCN 1-5 ppm) and lower and different percentages in other matrices such as, for example, in the percolate of a rubbish dump (H₂S 20-80 ppm; mercaptans 50-110 ppm; NH₃ 10-50 ppm: amines 20-80 ppm). In matrices treated with the compositions reported here below most of the bad-smelling gases were found to be absent after a period of contact depending from the matrices and gas concentrations and ranging from 10 minutes to two hours, except for animal excreta, such as those of pigs, in which the ammonia concentrations are very high.

1 Kg of pig excreta and 1 l of percolate are treated with 10 g of a solid composition constituted by HSO₅⁽⁻⁾ 0,24 % (Oxone® 0,66 %) and alkylarylsulfate sodium salt 0.33 % directly added to them. The above composition is used in solution to deodorize solid materials in amounts higher than HSO₅⁽⁻⁾ 0,0024 % (Oxone® 0,0066 %) and preferably in concentrations between HSO₅⁽⁻⁾ 0,024-0,24 % (Oxone® 0,066-066 %) using 1 l of solution for 1 m³ of materials (ex: solid urban wastes) or 1 l for about 100 m² of surface treated by a spraying system. The amount of solution used can be higher when the treatment is performed by a washing machine.

**Table 2**

| Results of deodorizing tests performed on a sample | | | | | | | | | |
|---|---|---|---|---|---|---|---|---|---|
| Compounds | Pig excreta | | | Percolate | | | Organic Wastes | | |
| (p.p.m.) | b.t. | a.t. | time | b.t. | a.t. | time | b.t. | a.t. | time |
| NH₃ | 80 | 0 | 30' | 30 | 0 | 30' | 20 | 0 | 5' |
| HCN | 2 | 0 | 10' | - | - | - | - | - | - |
| Mercaptans | 50 | 0 | 10' | 70 | 0 | 10' | 15 | 0 | 5' |
| H₂S | 40 | 0 | 10' | 50 | 0 | 10' | 40 | 0 | 5' |
| Amines | - | - | - | 40 | 0 | 10' | 20 | 0 | 5' |

| | | | | | | | | | |
|---|---|---|---|---|---|---|---|---|---|
| b.t.: before treatment; a.t.: after treatment; time: time of contact | | | | | | | | | |

### Test for the confirmation of selective action of composition on a natural matrix containing aerobic and anaerobic bacteria

To verify the selective oxidising action against ammonia, this was determined with a chemical stripping and assay method, together with Kjeldal nitrogen, BOD₅, COD and nitrates to assess the extent of the oxidation. To 100 g of fresh manure was added 1 g of the compositions against an untreated fresh manure (blank) and, after mixing, was left in contact for ten days. The samples were alkalinised with NaOH 6N and NH₃ was extracted by stripping with a nitrogen current and collected in a solution of H₂SO₄ 0.1N which was back-titrated with NaOH 0.1N. Nitrates were determined using the salicylate method Standard Methods of the examination of Water and Wastewater APHA-AWWA-WPCF. The treated samples showed a reduction in Kjeldal nitrogen which does not include the nitrates in its value, an increase in BOD₅ in the first two days with a reduction on day 5 only to rise again, showing that the oxygen released by the peroxymonosulphate is consumed in the 5 days and that the aerobic bacterial load is not impaired and is capable of proceeding with the oxidising processes on the organic substrates present (Figure 1). COD shows only a slight reduction, while the NH₃ concentration diminishes by approximately 40%, accompanied by an approximately 150% increase in nitrates.

The BOD₅ trend shown in Figure 1 has been confirmed in different aqueous matrices and is considered characteristic of the behaviour of the composition of the invention. As evident from Figure 1, the peroximonosulfate-compound acts in the aqueous medium containing the bacteria by producing an increase in the BOD₅ value during a period of 1-3 days, a subsequent decrease during a period of 3-5 days and a further increase of said value during a period of the following 6-8 days.

| | Kjeldal g/l N₂ | BOD₅ mg/l O₂ | COD mg/l O₂ | NH₃ g/l N₂ | NO₃-mg/l |
|---|---|---|---|---|---|
| Blank* | 1.30 | 850 | 2530 | 1.25 | 1.01 |
| Sample** | 0.60 | 590 | 2420 | 0.75 | 2.55 |

The experimental data demonstrate (i) that the peroxide supplies the aerobic bacterial flora with a greater amount of oxygen for oxidising the organic substrates, as confirmed by the reduction in Kjeldal nitrogen, (ii) that the compositions selectively inhibit the anaerobic bacterial flora responsible for the production of toxic and bad-smelling substances and (iii) that the compositions are also capable of oxidising ammonia, which is harder to oxidise, by converting it to nitrate. This mechanism characterises the compositions and distinguishes them from disinfectant mixtures that release chlorine and prove sterilising, completely eliminating both the anaerobic and aerobic bacterial load (US 4,822,512).

The second method was developed by selecting a number of subjects endowed with a particularly sensitive olfactory sensory system who verified the disappearance of the bad odour from the matrices after the latter had been treatment with the compositions. In some cases, as with the waters of animal residues or organic waste treatment plants, the time needed to eliminate the bad odour was just a few minutes.

### Example 1

a) Solid compositions were prepared with the components shown in Table 3.
   The solid compositions were preferably used at concentrations from 0.05% to 10% by weight in aqueous solutions prepared at the time of use, and their activity remained substantially unaltered for approximately seven days.
b) Liquid compositions were prepared with the components shown in Table 4.
   The liquid compositions were preferably used at concentrations from 0.05% to 10%, and their activity remained substantially unaltered for approximately seven days.

**Table 3**

| **Mixture n°** | **Peroxide %** | **Surfactant %** | **Zeolite %** | **Indicator %** | **Buffer %** | **pH** |
|---|---|---|---|---|---|---|
| 1 | HSO₅⁽⁻⁾ 80 | alkylbenzensulfonate 20 | | | | 2,5 |
| 2 | HSO₅⁽⁻⁾ 80 | alkylbenzensulfonate 20 | | amaranth 0,001 | | 2,5 |
| 3 | HSO₅⁽⁻⁾ 75 | alkylbenzensulfonate 20 | Clinoptilol ite 5 | | | 2,5 |
| 4 | HSO₅⁽⁻⁾ 75 | alkylbenzensulfonate 20 | A 5 | amaranth 0,001 | | 2,5 |
| 5 | HSO₅⁽⁻⁾ 80 | alkylbenzensulfonate 20 | | amaranth 0,001 | | 2,5 |
| 6 | HSO₅⁽⁻⁾ 69 | alkylbenzensulfonate 30 | | amaranth 0,001 | bicarbonte 0,1 | 7,0 |
| 7 | HSO₅⁽⁻⁾ 75 | alkylbenzensulfonate 20 | Clinoptilol ite 5 | amaranth 0,001 | | 2,5 |
| 8 | HSO₅⁽⁻⁾ 69 | alkylbenzensulfonate 25 | Clinoptilol ite 5 | amaranth 0,001 | bicarbonte 0,1 | 7,0 |
| 9 | HSO₅⁽⁻⁾ 75 | alkylbenzensulfonate 20 | A 5 | amaranth 0,001 | | 2,5 |
| 10 | HSO₅⁽⁻⁾ 69 | alkylbenzensulfonate 25 | A5 | amaranth 0,001 | bicarbonte 0,1 | 7,0 |
| 11 | Caro's reagent 65 | Sodium laurylsulfate 35 | | amaranth 0,003 | | 2,0 |
| 12 | Caro's reagent 63 | Sodium laurylsulfate 30 | Clinoptilol ite 8 | amaranth 0,003 | | 2,0 |
| 13 | Caro's reagent 65 | Sodium laurylsulfate 34,9 | | amaranth 0,003 | Potassium bicarbonte 0,1 | 7,0 |
| 14 | Caro's reagent 60 | Sodium laurylsulfate 30 | A 10 | amaranth 0,003 | | 2,0 |
| 15 | Caro's reagent 59 | Sodium laurylsulfate 30 | A 10 | amaranth 0,003 | Potassium bicarbonte 0,1 | 7,0 |
| 16 | peroxymonosulfate of ammonium 35 | Cetrimmonium chloride 65 | | amaranth 0,001 | | 2,5 |
| 17 | peroxymonosulfate of ammonium 35 | Cetrimmonium chloride 55 | Clinoptilol ite 10 | amaranth 0,001 | | 2,5 |
| 16A-17A | The same as 16-17 | The same as 16-17 | The same as 16-17 | The same as 16-17 | sodium bicarbonte 0,1 | 7,0 |
| 18 | Caro's reagent 35 | Cetrimmonium chloride 55 | Clinoptilol ite 10 | amaranth 0,001 | | 2,5 |
| 19 | Caro's reagent 35 | Cetrimmonium chloride 55 | A 10 | amaranth 0,001 | | 2,5 |
| 18A-19A | The same as 18-19 | The same as 18-19 | The same as 18-19 | The same as 18-19 | sodium bicarbonte 0,1 | 7,0 |

**Table 4**

| **Liquid Mixture n°** | **Oxidant %** | **Surfactant %** | **Zeolite %** | **Indicator %** | **Buffer %** | **pH** |
|---|---|---|---|---|---|---|
| 20 | Caro's reagent 65 | lauryl dimethylbenzylmmonium chloride 35 | | amaranth 0,001 | | 2,5 |
| 21 | Caro's reagent 61 | lauryl dimethylbenzylammonium chloride 31 | Clinoptilol ite 8 | amaranth 0,001 | | 2,5 |
| 20A-21A | The same as 20-21 | The same as 20-21 | The same as 20-21 | The same as 20-21 | sodium bicarbonte 0,15 | 7,0 |
| 22 | Peroxymonosulfate of ammonium 35 | lauryl dimethylbenzylammonium chloride 65 | | amaranth 0,001 | | 2,5 |
| 23 | peroxymonosulfate of ammonium 35 | lauryl dimethylbenzylammonium chloride 59 | A 6 | amaranth 0,001 | | 2,5 |
| 24 | Caro's reagent 65 | Diquat 35 | | amaranth 0,001 | | 2,5 |
| 25 | Caro's reagent 61 | Diquat 31 | Clinoptilol ite 8 | amaranth 0,001 | | 2,5 |
| 26 | peroxymonosulfate of ammonium 35 | Diquat 59 | A 6 | amaranth 0,001 | | 2,5 |
| 22A-26A | The same as 22-26 | The same as 22-26 | The same as 22-26 | The same as 22-26 | Potassium bicarbonte 0,1 | 7,0 |
| 1A-26A | The same as 1-26 | non ionic surfactants 20-70 | The same as 1-26 | The same as 1-26 | The same as 1-26 | 7,0 |
| 1A-26A | The same as 1-26 | non ionic surfactants 20-70 | Clinoptilol ite 5 | The same as 1-26 | The same as 1-26 | 2,5 |
| 1A-26A | The same as 1-26 | non ionic surfactants 20-70 | A 5 | The same as 1-26 | The same as 1-26 | 2,5 |
| 1A-26A | The same as 1-26 | non ionic surfactants 19-69 | The same as 1-26 | The same as 1-26 | Potassium bicarbonte 0,1 | 7,0 |
| 27(*) | potassium per carbonate 66 | Sodium laurilsulfate 33 | | | | 8 |
| 28(*) | hydrogen peroxide 50 | Sodium laurilsulfate 33 | | | | 5 |
| 29(*) | peroxiacetic acid 65 | Sodium laurilsulfate 33 | | | | 5 |

| | | | | | | |
|---|---|---|---|---|---|---|
| (*) Comparison - The Mixtures are not stable and develop gases rapidly and their activity disappears in a short time. Peracetic acid has a very low deodorizing activity and leaves a smell of acetic acid. | | | | | | |

### Example 2

Solid urban waste, hereinafter referred to as SUW, accumulated in reception halls before being separated and treated, is deodorized and sanitized by spraying it with a solution as in examples 1-5, 7, 9, 11-12, 14, 16-19, 20-26 and the same mixtures containing non ionic surfactants at concentrations of 0.5-2.0% by means of a nebulisation system using 1-3 l of solution per m³ of waste.

### Example 3

The SUW, sieved and ground, is conveyed to the composting hall where it is treated by spraying it at the exit points of the conveyor belts with a solution as in examples 1-5, 7, 9, 11-12, 14, 16-19, and the same mixtures containing non ionic surfactants at concentrations of 0.5-2.0% by means of a nebulisation system using 1-3 l of solution per m³ of waste. This treatment deodorizes the materials and activates the oxidative processes, supplying oxygen that facilitates fermentation, thus reducing the maturation time of the compost. The same type of treatment can be repeated on compost heaps if necessary and to deodorize and disinfect the halls.

### Example 4

The superficial organic materials present in the biofilters, when sprayed by means of nebulisation with a solution as in examples 1-5, 7, 9, 11-12, 14, 16-19, and the same mixtures containing non ionic surfactants at a concentration of 0.5-2.0%, are deodorized and their oxidation is facilitated.

### Example 5

The means of collection and transportation of SUW are deodorized and disinfected by means of washing with a solution as in examples 1-5, 7, 9, 11-12, 14, 16-19, 20-26 and the same mixtures containing non ionic surfactants at a concentration of 0.05-1.

### Example 6

Particular compositions are used for washing waste bins by means of waste bin washers. To the mixtures as in examples 1-3, 5, 8, 11, 15-16 and corresponding mixtures containing non ionic surfactants are added 100-400 g of surfactant already present in the compositions per 1 kg or 1 l of solution. These compositions are used in amounts of 1-2 kg or 1-2 l per m³ of water present in the tank of the waste bin washer (generally 5-6 m³). These mixtures, in addition to deodorizing and disinfecting the waste bins, exert a better washing action on the surfaces, facilitating the removal of solid or semisolid materials attached thereto.

### Example 7

The compositions as in examples 1-3, 5, 8, 11, 15-16 and corresponding mixtures containing non ionic surfactants, dissolved in washing water in amounts of 1-2 kg or 1-2 l per m³ of the machines used for washing and cleaning streets or areas in which food markets operate, particularly fish markets that emit very bad odours, rapidly deodorize, wash and disinfect the areas treated.

### Example 8

The accumulation of SUW in dumps or waste tips unfortunately remains one of the most widespread systems for waste disposal with a major environmental impact owing to the bad odour they emit in fermentation, which is even more marked in the percolate collecting on the impermeable base of the dump. The compositions as in examples 1-3, 5, 8, 11, 15-16 and corresponding mixtures containing non ionic surfactants at concentrations of 0.05-1.5% are used to spray the layers of SUW in an amount of 1-2 l per m³ of material by means of a system to be inserted in the compactors that compress the various layers. These compositions eliminate the bad odours and activate the aerobic attack processes on the organic materials present. In addition, the oxidising power of the mixtures favours the oxidation of toxic metals which, on going over to higher oxidisation states, are more easily subject to hydrolysis and can therefore precipitate more easily and remain adhering to the solid phase of the waste, thus reducing their concentration in the percolate.

Treatment of the percolate with the compositions as in examples 1-5, 7, 9, 11-12, 14, 16, and the same mixtures containing non ionic surfactants by direct dissolving therein at concentrations of 0.5-5% deodorizes and clarifies the water, inhibits the anaerobic fermentation processes with reduction of toxic gases, favours the processes of oxidation of the organic materials present and of toxic metals that can precipitate more rapidly at controlled pH and be separated more easily.

### Example 9

Increasingly widespread is the differentiated collection of waste due to the need to increase the recycling of materials and thus reduce the amount of discarded material. Conservation of the organic components present in all waste of domestic origin, the food industry, canteens and restaurants is a major problem, bearing in mind that they cannot be collected daily for reasons of cost. The compositions as in examples 1-5, 7, 9, 11-12, 14, 16, 21, 23, 25-26 and the same mixtures containing non ionic surfactant at concentrations of 0.05-1.5% used to spray organic materials of any origin by means of simple nebulisers deodorize and sanitize them, thus allowing their conservation for more than ten days. The treatment must be applied at each new addition of organic material to the mass already conserved and, if necessary, can be repeated several times.

### Example 10

The treatment modalities described in example 12 are also used to deodorize and disinfect animal residues produced by the meat processing industry, by slaughter-houses and butcher's shops, allowing problem-free conservation even for prolonged periods. In addition, the compositions can be used to wash, deodorize and disinfect the rooms of slaughter-houses and, in industries that process animal residues, can also be applied to animal residues in storage depots for processing and for the deodorising, washing and disinfection of the structures of the plant. For deodorizing and disinfecting the washing and effluent waters of slaughter-houses and plant treating animal residues, the compositions as in examples 1-5, 7, 9, 11-12, 14, 16, 21, 23, 25-26 and the same mixtures containing non ionic surfactants are used by direct dissolving therein at concentrations of 0.5-5%, which deodorize and clarify the waters and inhibit the anaerobic fermentation processes with reduction of toxic gases.

### Example 11

The compositions as in examples 1-5, 7, 9, 11-12, 14, 16-19, 20-26 and the same mixtures containing non ionic surfactants at concentrations of 0.05-2.0% are used for deodorizing, washing and disinfecting the stables and stalls of breeding farms for pigs, sheep, cattle, and horses and for the structures present in poultry breeding facilities using washing systems already existing in such structures. In the case of pig-breeding farms, the compositions must be added directly to the recycling waters from the primary excreta settling tanks. This system is used in plants to reduce the volumes of water to be used but also so as not to substantially increase the volumes of material to be stored in the collection tanks for the maturation of the excreta. The compositions which, through the washing waters, come into contact with the excreta facilitate their maturation, inhibit the growth of anaerobic bacteria and promote the oxidation of the bad-smelling and toxic gases present, including ammonia, the oxidation of which to nitrates is activated by the peroxides.

### Example 12

The compositions as in examples 6, 8, 10, 13, 15-26 and the same mixtures containing non ionic surfactants, in which the pH of the respective solutions is neutral, are preferably used at concentrations of 0.05-2.0% for household use for the deodorizing, washing and disinfection of delicate surfaces that may be attacked by a distinctly acidic pH, such as delicate floors, non-steel metal surfaces, baths or pet litters. The solutions are prepared directly in the nebulising canisters by pouring in the required amount of the compositions that are then dissolved or diluted by means of the addition of water up to a given value. In that way, the solution is already in the nebuliser and ready for use for spraying the surfaces to be treated. The compositions, on the other hand, are added direct to the amount of water needed for washing floors prior to use according to the scheduled dosages..

### Example 13

The treatment modalities reported in example 15 are preferably used for the deodorizing, washing and disinfection of refrigerator cells and food storage depots emitting particularly strong odours such as those of meat and fish.

## Claims

1. Use of a composition comprising peroximonosulfate, a surfactant and an oxidimetric colouring agent for the generation of the oxidizing couple H2O2/HSO5(-) on matrices to be treated with the composition and for the selective elimination of the anaerobic bacteria contained in the matrices to be treated with the composition so to deodorize the matrices.

## Patentansprüche

1. Verwendung einer Zusammensetzung umfassend Peroxymonosulfat, ein Tensid und ein oxidimetrisches Färbemittel zur Erzeugung des Oxidationspaares H2O2/HSO5(-) auf Matrizen, welche mit der Zusammensetzung zu behandeln sind und zur selektiven Eliminierung von anaeroben Bakterien, welche in mit der Zusammensetzung zu behandelnden Matrizen enthalten sind, um die Matrizen zu desodorieren.

## Revendications

1. Utilisation d'une composition comprenant du peroxymonosulfate, un tensioactif et un agent colorant oxydimétrique pour la génération du couple oxydant H2O2/HSO5(-) sur des matrices destinées à être traitées avec la composition et pour l'élimination sélective de bactéries anaérobies contenues dans les matrices destinées à être traitées avec la composition, afin de désodoriser les matrices.
